# EUROPÄISCHE PATENTSCHRIFT

(11) **EP 0 696 573 B1**
(45) Veröffentlichungstag und Bekanntmachung des Hinweises auf die Patenterteilung: **31.03.1999**
(21) Anmeldenummer: 95111997.3
(22) Anmeldetag: 31.07.1995
(51) Int. Cl.: C07C 209/36, C07C 211/44

(54) **Verfahren zur Herstellung von aromatischen Aminen**
Process for the preparation of aromatic amines
Procédé pour la préparation d'amines aromatiques

(30) Priorität: 08.08.1994 DE 4428017
(43) Veröffentlichungstag der Anmeldung: 14.02.1996
(73) Patentinhaber: BAYER AG, 51368 Leverkusen (DE)
(72) Erfinder: Langer, Reinhard, Dr., D-47800 Krefeld (DE); Buysch, Hans-Josef, Dr., D-47809 Krefeld (DE); Wagner, Paul, Dr., D-40597 Düsseldorf (DE); Pentling, Ursula, Dr., D-47906 Kempen (DE)

(56) Entgegenhaltungen:
- EP-A- 0 011 090
- DE-A- 1 809 711
- GB-A- 1 452 466
- GB-A- 2 182 330
- CHEMICAL ABSTRACTS, vol. 112, no. 4, 22.Januar 1990 Columbus, Ohio, US; abstract no. 26341t, Seite 362; Spalte 2; & CS-A-258 375 (PAVLAS, PAVEL ET AL.) 14.April 1989
- CHEMICAL ABSTRACTS, vol. 100, no. 16, 16.April 1984 Columbus, Ohio, US; abstract no. 123155v, GRAMATIKOV, K. ET AL. 'Adiabatic process of catalytic reduction of nitrobenzene to aniline.' Seite 108; Spalte 2; & IZV. KHIM., Bd. 16, Nr. 1-2, 1983 Seiten 38-43,
- CHEMICAL ABSTRACTS, vol. 117, no. 20, 16.November 1992 Columbus, Ohio, US; abstract no. 194013j, Seite 120; Spalte 2; & CS-A-263 430 (ROZINEK, RADOVAN ET AL.) 20.Januar 1992

## Beschreibung

Gegenstand der Erfindung ist ein Verfahren zur Hydrierung von Nitroaromaten zu aromatischen Aminen in der Gasphase an ortsfesten Katalysatoren, worin dem Katalysator von außen weder Wärme zugeführt noch Wärme entzogen wird, d.h., daß das Verfahren adiabat durchgeführt wird, und worin der umzusetzende Nitroaromat mit einem Vielfachen des daraus entstehenden aromatischen Amins sowie einem Vielfachen an Wasser und Wasserstoff unter Druck über den Katalysator geleitet wird.

Aromatische Amine sind wichtige Zwischenprodukte, welche preiswert und in großen Mengen zur Verfügung stehen müssen. Daher müssen z.B. für die Hydrierung von Nitrobenzol Anlagen mit sehr großen Kapazitäten gebaut werden.

Die Hydrierung von Nitroaromaten ist eine stark exotherme Reaktion. So werden bei 200°C bei der Hydrierung von Nitroxylol zu Xylidin ca. 488 kJ/mol (117 kcal/mol) und bei der Hydrierung von Nitrobenzol ca. 544 kJ/mol (130 kcal/mol) freigesetzt.

Die Abführung und Verwendung der Reaktionswärme ist demnach ein wichtiger Punkt bei der Durchführung von Verfahren zur Hydrierung von Nitroaromaten.

So wird in einer etablierten Verfahrensweise der Katalysator als fluidisiertes, thermostabilisiertes Bett betrieben (US 3 136 818). Der effektiven Wärmeabfuhr dieser Verfahrensweise stehen Probleme durch uneinheitliche Verweilzeitverteilung (Nitrobenzoldurchbruch) und Katalysatorabrieb gegenüber.

Enge Verweilzeitverteilungen und geringer Katalysatorabrieb sind in Reaktoren mit stationärer Katalysatorschüttung möglich. Jedoch ergeben sich in solchen Reaktoren Probleme mit der Thermostatisierung der Katalysatorbetten. Im allgemeinen werden thermostatisierte Rohrbündelreaktoren verwendet, welche, besonders bei großen Reaktoren, einen sehr aufwendigen Kühlkreislauf besitzen (DE-OS 2 201 528, DE-OS 3 414 714). Derartige Reaktoren sind komplex und verursachen hohe Investitionskosten. Mit der Baugröße rasch ansteigende Probleme bezüglich mechanischer Festigkeit und gleichmäßiger Thermostatisierung der Katalysatorschüttung machen große Aggregate solchen Typs unwirtschaftlich.

Einfache Reaktoren, wie sie für das weiter unten beschriebene erfindungsgemäße Verfahren verwendet werden, enthalten nur Katalysatorschüttungen und besitzen kein System zur Wärmehaushaltung im Reaktor. Sie sind leicht in einen technischen Maßstab zu übertragen, in allen Größen preiswert und robust. Die Reaktionsenthalpie spiegelt sich bei diesem Reaktortyp in der Temperaturdifferenz zwischen Edukt- und Produktgasstrom quantitativ wieder.

Für die stark exotherme Hydrierung von Nitroaromaten wird bisher weder die Verwendung solcher Reaktoren beschrieben, noch werden geeignete Katalysatoren und geeignete Betriebsweisen aufgezeigt.

GB 1.452.466 befaßt sich mit einem Verfahren zur Nitrobenzolhydrierung, bei dem ein adiabater Reaktor einem isothermen Reaktor nachgeschaltet ist. Dabei wird der größte Teil des Nitrobenzols in einem thermostatisierten Rohrbündelreaktor umgesetzt, lediglich die Hydrierung des Restgehaltes an Nitrobenzol erfolgt bei relativ geringem Wasserstoffüberschuß (kleiner 30:1) in einem adiabaten Reaktor.

Der Vorteil des völligen Verzichts auf einen thermostatisierten Reaktor bei rein adiabater Umsetzung wurde nicht erkannt.

DE-AS 1 809 711 befaßt sich mit dem gleichmäßigen Einbringen von flüssigen Nitroverbindungen in einen heißen Gasstrom durch Verdüsen, bevorzugt an verengten Stellen unmittelbar vor dem Reaktor. Auf den Aufbau des Reaktors wird in der Anmeldungsschrift nicht eingegangen. Dem Beispiel kann man jedoch entnehmen, daß trotz eines beachtlichen Wasserstoffüberschusses mindestens 34 % der Reaktionsenthalpie den Reaktor nicht mit dem Produktgas verlassen hat.

In DE-OS 36 36 984 wird ein Verfahren zur gekoppelten Produktion von Nitro- und Dinitroaromaten aus den entsprechenden Kohlenwasserstoffen durch Nitrierung und deren nachfolgende Hydrierung beschrieben. Die Hydrierung erfolgt in der Gasphase bei Temperaturen zwischen 176 und 343,5°C. Dem Beispiel ist zu entnehmen, daß der Wasserstoffstrom auch zur Reaktionswärmeableitung aus den Reaktoren dient. Es wird eine Apparatur zur Gasphasenhydrierung beschrieben, die im wesentlichen aus zwei hintereinander geschalteten Reaktoren mit Zwischenkühlung und Eduktzwischeneinspeisung besteht, auf deren Größe und Aufbau nicht eingegangen wird. Jedoch kann man dem Temperaturprofil der Reaktoren entnehmen, daß ein nicht unerheblicher Anteil der Reaktionswärme nicht mit dem Produktgasstrom den Reaktor verläßt. So besitzt der Reaktor Nr. 1 eine Eingangstemperatur von 181,7°C, eine heißeste Stelle von 315,6°C und eine Ausgangstemperatur von 277,2°C, der Reaktor Nr. 2 besitzt eine Eingangstemperatur von 203,9°C, eine heißeste Stelle von 300°C und eine Ausgangstemperatur von 296,7°C. Ob bei einer Umsetzung in technische Größenordnungen von z.B. 80.000 jato die Reaktoren eine Kühleinrichtung benötigen oder nicht, wird in DE-OS 36 36 984 nicht gesagt. Sowohl in DE-OS 36 36 984 als auch in DE-OS 18 09 711 wird nicht explizit auf die Problematik der Wärmeabfuhr bei Gasphasenhydrierungen eingegangen.

Daß neue, verbesserte Anlagen zur Nitrobenzolhydrierung zu Anilin in Betrieb genommen wurden, kann man der Zeitschrift "Hydrocarbon Processing 59" (Bd. 59, 1979, Heft 11, S. 136) entnehmen. Der Veröffentlichung ist zu entnehmen, daß die Dampfgewinnung und die Reaktion dabei in einem Verfahrensschritt eng gekoppelt durchgeführt werden.

In allen oben genannten Veröffentlichungen werden Cu-Katalysatoren eingesetzt, die ausschließlich mit niedrigen Belastungen (<0,1 g/ml h) und auf niedrigem Temperaturniveau betrieben werden. Daraus resultieren geringe Raumzeitausbeuten.

Neben den erwähnten Kupferkatalysatoren sind zahlreiche andere Kontakte für die Gasphasenhydrierung von Nitroaromaten beschrieben. Sie sind in vielen Publikationen beschrieben und umfassen als hydrieraktive Elemente und Verbindungen Pd, Pt, Ru, Fe, Co, Ni, Mn, Re, Cr, Mo, V, Pb, Ti, Sn, Dy, Zn, Cd, Ba, Cu, Ag, Au, zum Teil als Oxide, Sulfide oder Selenide und auch in Form einer Raney-Legierung sowie auf Trägern, wie Al₂O₃, Fe₂O₃/Al₂O₃, SiO₂, Silikaten, Kohle, TiO₂, Cr₂O₃.

Auch diese Katalysatoren werden mit nur geringen Belastungen in einem Temperaturbereich unterhalb 350°C beschrieben.

In keiner bisherigen Veröffentlichungen wird beschrieben, daß es vorteilhaft sei, eine größere Menge aromatisches Amin und Wasser mit der aromatischen Nitroverbindung über den Kontakt zu leiten.

Meist wird im Gegenteil reiner Wasserstoff mit reiner Nitroverbindung über den Katalysator geleitet. In DE 1 809 711 und DE 3 636 984 sind relativ zur eingespeisten Menge an Nitroverbindung allenfalls geringe Mengen aromatisches Amin im Edukt für den Reaktor. In DE 1 809 711 werden pro 1 000 g Nitrobenzol ca. 1 502 g Wasserstoff, aber nur 692 g Wasser und sogar nur 92,9 g Anilin in den Reaktor gefahren; dies spiegelt lediglich die Tatsache wieder, daß man Wasser und Anilin mit vertretbarem Aufwand nur unvollständig aus dem Kreisgas entfernen kann.

Man kann sagen, daß nach dem bisherigen Stand der Technik bei der Gasphasenhydrierung von Nitroaromaten immer nach folgendem Prinzip vorgegangen wurde: Verdampfen des Nitroaromaten, Leiten des Nitroaromaten mit einem Wasserstoffüberschuß über den Katalystar, möglichst vollständiges Abtrennen des entstandenen aromatischen Amins und Wasser vom Wasserstoffüberschuß, Recyclieren des Wasserstoffüberschusses.

Es wurde überraschenderweise gefunden, daß man zu einer besonders günstigen Verfahrensweise zur Gasphasenhydrierung von Nitroaromaten gelangt, welche es gestattet, mit einfachen Apparaturen, bei hohen Energieausbeuten und hohen Produktselektivitäten aromatische Amine herzustellen, wenn man große Mengen an aromatischem Amin und Wasser mit dem Kreiswasserstoff recycliert und hauptsächlich nur das gebildetete aromatische Amin und Reaktionswasser aus diesem Gaskreislauf auskreist.

Die Erfindung betrifft ein Verfahren zur Herstellung von aromatischen Aminen der Formel 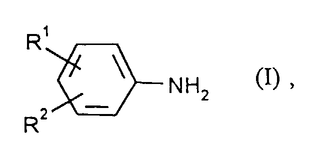 in der
- R¹ und R²: unabhängig voneinander Wasserstoff, Methyl oder Ethyl bedeuten, und
- R¹: zusätzlich Amino bedeuten kann,
durch Hydrierung von Nitroaromaten der Formel 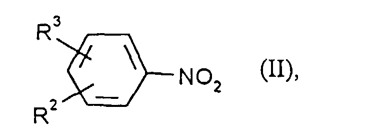 in der
- R² und R³: unabhängig voneinander Wasserstoff, Methyl oder Ethyl bedeuten, und R³ zusätzlich Nitro bedeuten kann,
in der Gasphase mit H₂ an ortsfesten Katalysatoren, das dadurch gekennzeichnet ist, daß bei einem Druck von 2 bis 50 bar, bevorzugt 3-20 bar, besonders bevorzugt 4-10 bar, und einer Temperatur im Bereich von 250-500°C, bevorzugt 290-470°C, besonders bevorzugt 320-440°C, ganz besonders bevorzugt 340-410°C, unter adiabatischen Bedingungen gearbeitet wird, wobei der Katalysator mit einem Kreisgas angeströmt wird, das pro Mol Nitrogruppen 3-150 Mol Wasserstoff, 2-100 Mole aromatische Aminogruppen und pro Mol aromati scher Aminogruppen 2-6 Mol, bevorzugt 2,1-4 Mol, besonders bevorzugt 2,2-3 Mol Wasser enthält, die pro Zeiteinheit gebildete Menge Amin und wasser auskondensiert wird und das restliche Kreisgas, gegebenenfalls nach Abtrennung eines Reinigungsstromes, recyclisiert und mit verdampftem Nitroaromat und mit Frischwasserstoff angereichert wird.

Die Nitrogruppen befinden sich ausschließlich in Eduktmolekülen, die aromatischen Aminogruppen ausschließlich in Produktmolekülen.

In bevorzugter Weise werden Nitroaromaten der Formel 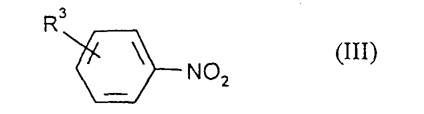 eingesetzt, in der
- R³: Wasserstoff, Methyl oder Ethyl bedeutet.

In besonders bevorzugter Weise wird Nitrobenzol eingesetzt.

Eine erfindungsgemäße Produktionsanlage besteht aus mindestens einem adiabaten Reaktor mit stationärem Katalysator. Es werden maximal 10, bevorzugt maximal 5, besonders bevorzugt maximal 3 solcher Reaktoren hintereinander geschaltet. Jeder in Reihe geschaltetete Reaktor kann durch mehrere parallel geschaltete ersetzt werden. Es werden maximal 5, bevorzugt maximal 3, besonders bevorzugt maximal 2 Reaktoren im Ersatz des einen Reaktors parallel geschaltet. Das erfindungsgemäße Verfahren kann demnach maximal 50 und minimal einen Reaktor beinhalten.

Eine größere Zahl von Reaktoren mit einer Katalysatorschüttung kann durch eine kleine Zahl mit mehreren Katalysatorschüttungen ersetzt werden.

Die Reaktoren bestehen aus einfachen Behältern mit isolierten Katalysatorschüttungen, wie sie z.B. in Ullmanns Encyclopedia of Industrial Chemistry (Fifth, Completely Revised Edition, Vol. B4, Seite 95-102, Seite 210-216) beschrieben werden.

Die Katalysatorschüttungen werden nach dem Stand der Technik auf oder zwischen gasdurchlässigen Wandungen angebracht. Insbesondere bei dünnen Schüttungen werden oberhalb, unterhalb oder oberhalb und unterhalb der Schüttung technische Vorrichtungen zur gleichmäßigen Gasverteilung angebracht. Dies können Lochplatten, Glockenböden, Ventilböden oder andere Einbauten sein, die durch Erzeugung geringen, aber gleichmäßigen Druckverlusts einen gleichförmigen Eintritt des Gases in die Katalysatorschüttung bewirken, bevorzugt werden Metall- oder Keramiksintermatten benutzt, wie sie z.B. die Firma Krebsöge vertreibt.

Statt Katalysatorschüttungen können auch als Trägermaterial geeignete Packungen verwendet werden. Dies wären z.B. Wabenkörper oder gewellte Schichten, wie sie von der Firma Sulzer unter dem Namen Katapak angeboten werden. Diese Packungen werden zum erfindungsgemäßen Einsatz natürlich vor dem Einbringen in den Reaktor durch das Aufbringen geeigneter Metallverbindungen aktiv gemacht.

Vor jeder Katalysatorschüttung wird frischer Nitroaromat in den Kreisgasstrom, der hauptsächlich aus recyclisiertem und frisch zugesetztem Wasserstoff sowie aus recyclisiertem aromatischen Amin und Wasser besteht, eindosiert. Das kann so geschehen, wie in DE 1 809 711 beschrieben, bevorzugt wird jedoch der Nitroaromat im Frischwasserstoff vollständig verdampft und dann gasförmig in den Kreisgasstrom gegeben. Der Vorteil dieser Verfahrensweise liegt in der deutlich geringeren Bildung von Ablagerungen im Reaktor und in den Zuleitungen. Die Verdampfung kann nach dem Stand der Technik in bekannten Verdampfern erfolgen, wie z.B. Fallfilm-, Steigrohr-, Einspritz-, Dünnschicht-, Umlauf- und Wendelrohrverdampfern. Bevorzugt wird in Fallfilm- und Einspritz-, ganz besonders bevorzugt in Fallfilmverdampfern verdampft. Weiterhin ist die Verdüsung des flüssigen Nitroaromaten in den Frischwasserstoff- oder Kreisgaswasserstoffstrom mittels Einstoff- oder Zweistoffdüsen möglich, wobei die Vereinigung des Eduktgasstromes nach einer Überhitzung in einem Wärmetauscher erfolgen kann. Der Verdampfung kann eine grundsätzlich bekannte Tröpfchenabscheidung nachgeschaltet werden. Der Eduktgasstrom wird in bekannter Weise mittels entsprechender Zuführung und Verteilung und oder durch Vermischungseinrichtungen, wie z.B. Mischern vom Typ SUX oder SMV der Firma Sulzer oder Kenics im Kreisstrom vermischt.

Nachj eder Katalysatorschüttung kann das sie verlassende Produktgas unter Dampfgewinnung abgekühlt werden. Dazu leitet man es durch einen oder mehrere Wärmetauscher. Dies können die dem Fachmann bekannten Wärmetauscher, wie z.B. Rohrbündel-, Platten-, Ringnut-, Spiralstrom-, Rippenrohrwärmetauscher sein.

Nach Verlassen eines jeden Wärmetauschers zur Umwandlung von Reaktionswärme in Dampf kann ein Teilstrom des Kreisgases abgezweigt und weiter abgekühlt werden, um gebildetes aromatisches Amin und gebildetes Wasser abzutrennen. In bevorzugter und besonders wirtschaftlicher Art erfolgt dies nach Verlassen des letzten Wärmetauschers.

Der Kreisgasstrom durchläuft daraufhin einen Kompressor, um den Strömungswiderstand von Reaktoren und Wärmeaustauschern auszugleichen und den Massenstrom des Kreisgases zu steuern.

Die Kompressoren können einfache, bekannte Apparate (z.B. Gebläse) sein, da der Druckverlust durch die Bauweise der Reaktoren kleingehalten werden kann, bevorzugt werden trockenlaufende Radial- oder Axialverdichter benutzt.

Der ausgekreiste Kreiswasserstoff kann prinzipiell an jeder beliebigen Stelle wieder in den Kreisgasstrom eingepeist und damit recyclisiert werden, ebenso wie das aromatische Amin und das Reaktionswasser an jeder beliebigen Stelle vor einer Nitroaromat-Einspeisung und nach einem Reaktor zusammen mit Wasserstoff ausgekreist werden kann, bevorzugt wird jedoch wie oben angegeben verfahren.

In einer speziellen Ausführungsform wird vor dem ersten Reaktor der von aromatischem Amin und Reaktionswasser weitgehend befreite ausgekreiste Wasserstoff in den Kreisgasstrom wieder eingeleitet (recyclisiert).

Der Gasstrom vor jeder Katalysatorausschüttung ist möglichst homogen und enthält pro Mol Nitrogruppen 2 bis 100 Mol aromatische Aminogruppen, bevorzugt 2,5 bis 50 Mol aromatische Aminogruppen, besonders bevorzugt 3 bis 20 Mol aromatische Aminogruppen, ganz besonders bevorzugt 3,5 bis 10 Mol aromatische Aminogruppen.

Pro Mol Aminogruppen enthält der Kreisgasstrom 2 bis 6 Mol Wasser, bevorzugt 2,1 bis 4 Mol Wasser, besonders bevorzugt 2,2 bis 3 Mol Wasser.

Pro Mol Nitrogruppen enthält der Gasstrom vor jeder Katalysatorschüttung 3-150 Mol Wasserstoff, bevorzugt 6-125 Mol Wasserstoff, besonders bevorzugt 12-100 Mol Wasserstoff, ganz besonders bevorzugt 25-75 Mol Wasserstoff.

Das Kreisgas steht unter Druck. Der Absolutdruck der Kreisgase liegt bei 2 bis 50 bar, bevorzugt bei 3-20 bar, besonders bevorzugt bei 4-10 bar.

Die Temperatur des Kreisgases liegt im Bereich von 250 und 500°C, bevorzugt 290-480°C, besonders bevorzugt 320-440°C, ganz besonders bevorzugt 340-410°C. Infolge der adiabaten Fahrweise steigt die Temperatur beim Reaktordurchgang.

Der zur Abtrennung von gebildetem aromatischen Amin und Reaktionswasser ausgekreiste Gasstrom wird auf eine Temperatur zwischen 80 und 20°C bevorzugt 60-25°C, besonders bevorzugt 40-30°C abgekühlt.

Das Kondensat wird in eine technische Einrichtung zur Trennung flüssiger Phasen geleitet und die wäßrige und die organische Phase getrennt aufgearbeitet.

Aus der wäßrigen Phase gewonnenes aromatisches Amin wird der Aufarbeitung der organischen Phase zugeführt. Die Aufarbeitungen erfolgen in bekannter Weise durch Destillation bzw. durch Strippen mit Wasserdampf.

Vom abgetrennten Kreisgaswasserstoff wird eine gewisse Menge zur Ausschleusung gasförmiger Verunreinigungen abgetrennt. Der Rest wird wieder aufgewärmt, komprimiert und in den Kreisgasstrom zurückgeführt. Auf diese Rückführung kann gegebenenfalls verzichtet werden.

Die Dicke der Katalysatorschüttungen kann zwischen 1 cm und 5 m, bevorzugt zwischen 5 cm und 2 m, besonders bevorzugt zwischen 10 cm und 1 m, ganz besonders bevorzugt zwischen 30 cm und 60 cm liegen.

Als Katalysatoren können alle bisher für die Gasphasenhydrierung von Nitroverbindungen beschriebenen Kontakte eingesetzt werden. Diese enthalten die Elemente der sogenannten Nebengruppen des Periodensystems der Elemente, entweder als Metalle oder Legierungen oder als Mischoxide und gegebenenfalls auf inertem Trägermaterial.

Als Trägermaterialien kommen in Frage: α- und γ-Al₂O₃, SiO₂, TiO₂, Roterde und Limonit, Fe₂O₃/Al₂O₃-Mischungen, CuO/Cr₂O₃-Mischungen, Wasserglas, Graphit, Aktivkohle (BET 20-100 m²/g) und Kohlefasern. Es können aber prinzipiell auch andere Träger eingesetzt werden.

Bevorzugt werden die Katalysatoren aus DE 2 849 002 eingesetzt. Dies sind Trägerkatalysatoren auf inerten Trägern mit einer BET-Oberfläche von weniger als 20 mg²/g, bzw. α-Al₂O₃ mit einer BET von weniger als 10 m²/g. Die in DE-OS 2 849 002 beschriebene Vorbehandlung mit einer Base ist nicht unbedingt erforderlich.

Auf dem Trägermaterial sind drei Aktivstoffklassen niedergeschlagen:
(a) 1 - 100 g/l Katalysator eines oder mehrerer Metalle der Gruppen VIIIa, Ib und IIb des Periodensystems der Elemente (Mendeljew),
(b) 1 - 100 g/l eines oder mehrerer Übergangsmetalle der Gruppen IIb, IVa, Va und VIa und
(c) 1 - 100 g/l eines oder mehrerer Hauptgruppenelemente der Gruppen IVb und Vb.

Elemente der Gruppe IIb können somit als Aktivstoffe (a) und (b) wirken. Bevorzugte Aktivstoffe sind Pd als Metall (a), V, Nb, Ta, Cr, Mo, W, Ti als Übergangsmetall (b) und Pb und Bi als Hauptgruppenelemente (c).

Besonders bevorzugt werden
(a) 20 bis 60 g Pd,
(b) 20 bis 60 g V und
(c) 10 bis 40 g Pb auf den Träger gebracht.

Die Aktivstoffe werden in Form ihrer löslichen Salze auf den Träger gebracht, gegebenenfalls sind mehrere Behandlungen (Tränkungen) pro Komponente erfordelrich. In bevorzugter Weise werden die Aktivstoffe nur schalenförmig, d.h. in der Nähe der Oberfläche des Katalysators aufgebracht.

Diese Kontakte werden in einem Temperaturbereich betrieben, der zwischen der Eingangstemperatur des Eduktgases und max. 500, bevorzugt maximal 480, besonders bevorzugt maximal 460, ganz besonders bevorzugt maximal 440°C liegt.

Der Metallgehalt dieser Katalysatoren kann beim erfindungsgemäßen Verfahren deutlich höher sein als in DE 2 849 002 beschrieben ist, dabei zeigen Katalysatoren mit großem Edelmetall gehalt überraschenderweise besonders hohe Selektivitäten. Der Gehalt an Pd liegt bei diesen Kontakten minimal bei 1 g und maximal bei 100, bevorzugt bei 60, besonders bevorzugt bei 40, ganz besonders bevorzugt bei 20 g Metall pro 1 Trägermaterial. V und Pb können im gleichen Maße verstärkt aufgebracht werden.

Weitere bevorzugte Katalysatoren sind solche, die Pd alleine oder mit Rh und/oder Ir und/oder Ru auf Kohleträgern mit geringer BET-Oberfläche tragen. Solche Trägermaterialien sind graphithaltig, es sind Graphite selbst und Kokse, wie Nadelkoks oder Petrolskoks. Diese Träger haben eine BET-Oberfläche von 0,2-10 m²/g. Verwendung finden erfindungsgemäß Katalysatoren, die auf Graphit oder graphithaltigem Koks als Träger 0,001-1,5 Gew.-% Pd, bezogen auf das Gesamtgewicht des Katalysators, enthalten, wobei das Pd zu 0 - 40 Relativprozent seiner Menge durch Ir und/oder Rh und/oder Ru ersetzt sein kann. Diese Katalysatoren enthalten also das (die) Edelmetall(e) in folgenden Anordnungen auf dem Träger: Pd allein, Pd/Ir, Pd/Rh, Pd/Ru, Pd/Ir/Rh, Pd/Ir/Ru, Pd/Rh/Ru, Pd/Ir/Rh/Ru. In vielen Fällen werden eine der genannten Zweierkombinationen oder Pd allein eingesetzt. In bevorzugter Weise liegt in den Katalysatoren auf Kohleträgern das Palladium in einer Menge von 0,005-1 Gew.-%, bevorzugt 0,05-0,5 Gew.-%, bezogen auf das Gesamtgewicht des Katalyators, vor. Die Untergrenze Null der Relativprozente der anderen genannten Platinmetalle zeigt den Gebrauch von Pd alleine an. Falls die anderen Platinmetalle eingesetzt werden, beträgt ihr Anteil bevorzugt insgesamt 10-40 Relativprozent; untereinander beträgt ihr Gewichtsverhältnis 1:1-3:1 zwischen je zweien.

Es hat sich weiterhin als vorteilhaft erwiesen, die genannten Katalysatoren zusätzlich mit einer schwefelhaltigen oder phosphorhaltigen, bevorzugt phosphorhaltigen Verbindungen zu dotieren. Ein solcher zusätzlicher Gehalt an Dotierungsmittel beträgt 0,1-2 Gew.-%, bevorzugt 0,1-1 Gew.-% Schwefel oder Phosphor, bevorzugt Phosphor, in chemisch gebundener Form, bezogen auf das Gesamtgewicht des Katalysators. Als phosphorhaltige Verbindungen für die Dotierung der erfindungsgemäßen Katalysatoren sind bevorzugt zu nennen: die Sauerstoffsäuren des Phosphors H₃PO₄, H₃PO₃, H₃PO₂ oder deren Alkalisalze, wie z.B. Natriumdihydrogenphosphat, Natrium- oder Kaliumphosphat oder Natriumhypophosphit.

Zur Herstellung der Katalysatoren auf Kohleträgern kann so vorgegangen werden, daß auf einen der genannten Träger in Form von Pillen, Kugeln, Stranggranulat oder Bruchstücken von etwa 1-10 mm Abmessung die genannten Edelmetalle in Form geeigneter Salze sowie die schwefelhaltige oder phosphorhaltige Verbindung in getrennten Arbeitsgängen aufgetragen werden, wobei nach jedem Auftrag getrocknet wird. Das Trocknen geschieht in bekannter Weise, beispielsweise bei 100-140°C und vermindertem bis normalem Druck, beispielsweise bei 1-1.000 mbar; als verminderter Druck kommt beispielsweise der einer Wasserstrahlpumpe in Betracht. Zum Tränken des Trägers können wäßrige Lösungen verwendet werden. Dies ist in bevorzugter Weise bei den schwefel- oder phosphorhaltigen Verbindungen, von denen wasserlösliche bevorzugt werden, der Fall. Die Edelmetallsalze werden jedoch bevorzugt in organischen Lösungsmitteln, wie einfachen Alkoholen, Ketonen, cyclischen Ethern oder Nitrilen, gelöst und aufgetragen. Beispiele für solche organischen Lösungsmittel sind Methanol, Ethanol, Propanol, Isopropanol, Aceton, Methyl-ethylketon, Dioxan, Acetonitril und vergleichbare Lösungsmittel. Bei Salzen mit organischen Anionen können auch Methylenchlorid und vergleichbare Lösungsmittel eingesetzt werden. Geeignete Salze der Edelmetalle sind beispielsweise ihre Chloride, Nitrate oder Acetate.

Nach dem Imprägnieren und dem abschließenden Trocknen steht der Katalysator zur Verfügung. Er wird in bevorzugter Weise im Reaktor vor Beginn der Hydrierung von Nitrolbenzol durch eine Behandlung mit Wasserstoff bei erhöhter Temperatur aktiviert. Eine solche erhöhte Temperatur liegt beispielsweise im Bereich von 200-400°C, bevorzugt im Bereich von 230-370°C.

Die genannten Katalysatoren sind in hervorragender Weise zur Hydrierung von Nitrobenzol zu Anilin verwendbar.

Bei Aktivitätsabfall des eingesetzten Katalysators kann er leicht in situ, d.h. im Hydrierreaktor, regeneriert werden. Hierzu wird der Katalysator bei 350-400°C nacheinander mit Wasserdampf, einem Stickstoff/Luft-Gemisch oder atmosphärischer Luft und schließlich Stickstoff behandelt. Die Behandlung mit Wasserdampf kann 1 bis 3 Stunden, die Behandlung mit Luft bzw. dem Stickstoff/Luft-Gemisch kann 1 bis 4 Stunden lang erfolgen. Eine solche Regenerierung ist nicht möglich bei Edelmetallkatalysatoren auf anderen als den beschriebenen Kohleträgern, z.B. bei aktivierter Kohle als Träger, da eine aktivierte Kohle bei einer solchen Regenerierung zu verbrennen beginnt. Zur erneuten Aktivierung des Katalysators kann sich eine Behandlung mit Wasserstoff bei 200-400°C anschließen.

Diese Katalysatoren werden im Temperaturbereich unter 480°C, bevorzugt unter 460°C, ganz besonders bevorzugt unter 440°C betrieben.

Die als bevorzugt beschriebenen Kontakte ermöglichen eine besonders lange Laufzeit zwischen den Regenerierungen.

Prinzipiell können die Katalysatorkörner jede beliebige Form aufweisen, wie z.B. Kugeln, Stäbchen, Raschigringe oder Granulat oder Tabletten. Bevorzugt werden Formkörper benutzt, deren Schüttungen einen niedrigen Stromungswiderstand bei guten Gas-Oberflächen-Kontakt aufweisen, wie z.B. Raschigringe, Sattelkörper, Wagenräder und Spiralen.

Das erfindungsgemäße Verfahren erlaubt besonders vorteilhafte Durchführungen der Gasphasenhydrierung, die zu konstant hohen Selektivitäten an aromatischem Amin und langen Katalysatorstandzeiten zwischen den Regenerationen des Katalysators führen, die i.a. im Abbrennen von kohlenstoffreichen Ablagerungen bestehen.

Eine Vorgehensweise besteht darin, daß bei konstantem Druck gearbeitet wird, und daß der Katalysator mit besonders hoher Belastung angefahren wird. Die Belastung wird dann im Zuge der Katalysatordesaktivierung während der Laufzeit zwischen zwei Regenerierungen so zurückgenommen, daß kein Nitroaromat durchbricht.

Eine andere gleichwertige Methode besteht darin, die Belastung des Katalysators konstant zu halten und mit niedrigem Systemdruck zu beginnen; kurz bevor Nitroaromat durchzubrechen beginnt, wird der Systemdruck langsam gesteigert.

Es kann auch eine Fahrweise zwischen den beiden Extremen konstanter Druck und konstante Belastung gewählt werden. Bevorzugt wird mit niedrigem Druck und niedriger Belastung angefahren, um dann im Verlauf der Katalysatordesaktivierung beide Größen zu steigern.

Die Belastung der Katalysatoren kann beim erfindungsgemäßen Verfahren sehr hoch sein und von 0,1 g bis zu 20 g Nitroaromat pro ml Kat. und Stunde, bevorzugt bis zu 15 g/ml·h, besonders bevorzugt bis zu 10 g/ml·h, ganz besonders bevorzugt bis zu 5 g/ml·h betragen.

Das erfindungsgemäße Verfahren zeichnet sich demnach durch hohe Raumzeitausbeuten aus, verbunden mit einer Verringerung der Apparategrößen. Das erfindungsgemäße Verfahren erlaubt ferner einfache Standard-Apparaturen zu benutzen und ermöglicht hohe Anlagenkapazitäten bei geringen Investitionskosten.

Der Gaskreislauf zur Abfuhr der Reaktionswärme vom Katalysator in die Dampferzeuger durchläuft keine Anlagenteile zur Ausschleusung der Reaktionsprodukte und muß demnach nicht soweit abgekühlt werden, daß Reaktionsprodukt auskondensiert. Dadurch sind nur kleine Wärmetauscheroberflächen nötig. Bauteile und Rohrleitungen, die den großen Kreisgasstrom zu bewältigen haben, reduzieren sich auf die Reaktoren, die Hochtemperaturwärmetauscher und den Kompressor.

Die Anlage kann mit geringem Druckabfall und damit mit großem Kreisgasumlauf und damit mit kleinem Temperaturanstieg im Katalysatorbett betrieben werden.

Das führt dazu, daß die Reaktionswärme auf hohem thermischem Niveau gewonnen werden kann, was die lukrative Gewinnung von hochgespanntem Dampf zur Stromerzeugung ermöglicht.

Dies führt zu einer spürbaren Verbesserung der Wirtschaftlichkeit des Verfahrens.

Die hohen Temperaturen führen zusammen mit der Verdünnung der aromatischen Nitroverbindung mit dem aromatischen Amin, dem Wasser und dem Wasserstoff und der Druck- bzw. Belastungs- bzw. Druck-Belastungs-Gradienten-Fahrweise zu einer erstaunlich hohen Selektivität über den gesamten Produktionszyklus zwischen zwei Katalysatorregenererierungen durch Abbrennen.

Das Verfahren ist besonders geeignet, um Nitrobenzol zu Anilin umzusetzen. Hierbei wird Anilin in durchgehend hoher Selektivität gebildet; dies liegt offenbar daran, daß in keinem Katalysatorvolumen das Verhältnis von Nitrogruppen zu aromatischen Aminogruppen größer als 0,5 ist.

### Beispiel 1

400 g Graphitgranulat EG 17 der Firma Ringsdorf, Granulat 1-3 mm, Rüttelgewicht 650-1000 g/l, BET = 0,3-0,4 m²/g) mit einer Saugfähigkeit von 7 ml Acetonitril pro 100 g Träger wurden in einem drehbaren Gefäß vorgelegt und mit einer Lösung aus 1,66 g Palladium-II-acetat in 26 g Acetonitril versetzt. Die Mischung wurde durch Drehen bis zur völligen Aufnahme der Lösung vom Trägermaterial bewegt. Danach erfolgte eine fünfminütige Trocknung des Feststoffes in einem starken aufsteigenden 40°C warmen Luftstrom. Die Tränkung mit Palladiumacetat in Acetonitril mit anschließender Trocknung wurde noch zweimal wiederholt. Der getrocknete Katalysator wurde anschließend 3 Stunden in einem 100°C heißem Wasserstoffstrom reduziert.

### Beispiel 2

5000 ml α-Al₂O₃ der Firma Condea (α-Tonerde, Dichte 1,02 g/ml, Kugeln mit einem Durchmesser von 1 mm, BET = 4 m²/g) mit einer Saugfähigkeit von 33,4 ml Wasser pro 100 g Träger wurden in einem drehbaren Gefäß vorgelegt und mit einer Lösung aus 553 g Dinatrium-tetra-chloropalladat in 1200 g Wasser versetzt. Die Mischung wurde solange durch Drehen vermischt, bis die gesamte Lösung vom Trägermaterial aufgenommen worden war. Danach erfolgte eine 10minütige Trocknung des Feststoffes in einem starken aufsteigenden 40°C warmen Luftstrom. Der getrocknete Katalysator wurde 3 Stunden bei 350°C mit Wasserstoff reduziert, der reduzierende und getrocknete Feststoff anschließend bei Raumtemperatur mit einer Lösung aus 500 g Oxalsäuredihydrat und 178,6 g Vanadiumpentoxid in 1030 g Wasser versetzt und solange durch Drehen vermischt bis die gesamte Lösung vom Trägermaterial aufgenommen worden war. Danach erfolgte eine 10minütige Trocknung des Feststoffes in einem starken aufsteigenden 40°C warmen Luftstrom, worauf eine erneute Tränkung mit der gleichen Menge einer Vanadiumoxalat-Lösung mit anschließender Trocknung im warmen Luftstrom folgte. Der getrocknete Katalysator wurde 4 Stunden bei 300°C getempert und dann auf Raumtemperatur abgekühlt. Es folgte eine Tränkung des Feststoffes, wie oben beschrieben, mit einer Lösung aus 128,2 g Blei-II-acetat-trihydrat in 1200 g Wasser. Danach wurde der Feststoff wiederum in einem starken aufsteigenden 40°C warmen Luftstrom 10 Minuten getrocknet, der getrocknete Katalysator 3 Stunden bei 350°C mit Wasserstoff reduziert und abschließend bei Raumtemperatur mit destilliertem Wasser gewaschen, bis das Waschwasser einen pH-Wert von 7 aufwies. Der so erhaltene Katalysator erfuhr eine abschließende 10minütige Trocknung in einem stark aufsteigenden Luftstrom bei 40°C.

### Beispiel 3

30 ml (31 g) des in Beispiel 1 hergestellten Katalysators wurden in eine druckfeste Apparatur, bestehend aus drei ölbeheizten Verdampfer-Überhitzern, einem isolierten elektrisch begleitbeheizten Reaktionsrohr, einem Kondensator, einem Auffanggefäß für das flüssige Produkt mit automatischer standgeregelter Austragung und einem Tröpfchenabscheider, gefüllt.

Der Innendurchmesser des Reaktionsrohres betrug 14,9 mm, die Wandstärke 3,2 mm. Das Reaktionsrohr war von oben nach unten in Strömungsrichtung auf eine Länge von 300 mm durch Kupferkerne (⌀ᵢ = 24 mm, ⌀ₐ = 44 mm) mit einem guten Wärmeübergang zur elektrischen Begleitheizung und darunter im Katalysatorbereich auf eine Länge von 500 mm durch eine 10 mm dicke Schicht Isolierband mit einem schlechten Wärmeübergang zur elektrischen Begleitheizung versehen.

Die Katalysatorüberschüttung war 180 mm lang und entlang der Rohrachse mit einem hohlen Metallröhrchen (⌀ₐ = 4 mm) zur Führung eines Thermoelementes versehen. Oberhalb und unterhalb der Katalysatorschüttung befanden sich Schüttungen aus 3 mm Maschendrahtringen zur Fixierung des Katalysatorbettes.

Die Apparatur wurde durch die drei Verdampfer-Überhitzer gleichmäßig mit einem konstanten Wasserstoffstrom bei einem definierten Druck beaufschlagt. In die Verdampfer wurde über Dosierpumpen getrennt Wasser, Anilin und Nitrobenzol gepumpt.

Nach vollständiger Verdampfung im Wasserstoffstrom wurden die Edukt-"Kreisgasströme" vereint und über den Kontakt geleitet.

Das Anfahren der Anlage erfolgte, indem bei 4 bar 460 Nl Wasserstoff eingeleitet, die ölthermostatisierten Verdampfer-Überhitzer auf 300°C und das Reaktionsrohr mit dem Katalysator auf 320°C aufgeheizt wurden.

Nach Erreichen der Solltemperaturen wurde die Anilindosierung auf 182 g (2 mol) und die Wasserdosierung auf 36 g (2 mol) pro Stunde eingestellt.

Daraufhin wurde begonnen 32,6 g (0,27 mol) Nitrobenzol pro Stunde in den dritten Verdampfer zu fördern.

Die Katalysatortemperatur, welche vorher entlang der Rohrachse einheitlich 320°C betrug, stieg mit Beginn der Nitrobenzolförderung rasch bis auf ca. 439°C an.

Das flüssige Produktgemisch bestand aus einer wäßrigen und einer organischen Phase. Die organische Phase enthielt zu 98,5 % Anilin, mit 0,5 % Cyclohexylanilin und 0,6 % Diphenylamin verunreinigt, und wurde als "Anilin" mit 182 g pro Stunde in den Anilinverdampfer zurückgefahren.

Die wäßrige Phase bestand fast ausschließlich aus Wasser und wurde verworfen.

Nach 3 Stunden Laufzeit lieferte die Anlage bereits 99,5%iges Anilin und nach 23 Stunden war das erhaltene Anilin 99,8%ig.

Mit diesesr Reinheit wurde das Anilin weitere 1000 Stunden ohne Anzeichen einer beginnenden Desaktivierung erhalten, worauf der Versuch abgebrochen wurde.

Die Nachweisgrenze der verwendeten GC-Analytik betrug ca. 5 ppm.

Die Nebenprodukte umfassen ca. 16 Verbindungen, die zusammen ca. 0,2 % der Produktmenge ausmachen.

### Beispiel 4

In die in Beispiel 3 beschriebene Apparatur wurden 30 ml des in Beispiel 2 beschriebenen Katalysators gefüllt und die Anlage, wie in Beispiel 3 beschrieben, betrieben.

Der Katalysator lieferte im wesentlichen das gleiche Hydrierungsprodukt wie der Katalysator auf Kohlenstoffträger.

Er erreichte 24 Stunden eine etwas bessere Anilinreinheit von 99,9 %, verglichen mit Beispiel 3, zeigte aber nach 1000 Stunden Laufzeit mit ca. 2 ppm Nitrobenzol im Produkt eine beginnende Desaktivierung.

## Patentansprüche

1. Verfahren zur Herstellung von aromatischen Aminen der Formel 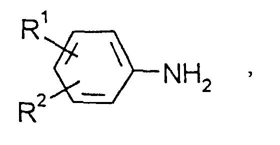 in der
R¹ und R² unabhängig voneinander Wasserstoff, Methyl oder Ethyl bedeuten,
und R¹ zusätzlich Amino bedeuten kann,
durch Hydrierung von Nitroaromaten der Formel 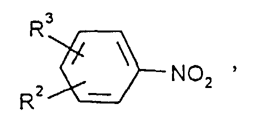 in der
R² und R³ unabhängig voneinander Wasserstoff, Methyl oder Ethyl bedeuten und zusätzlich Nitro bedeuten kann,
in der Gasphase mit H₂ an ortsfesten Katalysatoren, dadurch gekennzeichnet, daß bei einem Druck von 2 bis 50 bar, bevorzugt 3-25 bar, besonders bevorzugt 4-18 bar, und einer Temperatur im Bereich von 250-500°C, bevorzugt 290-480°C, besonders bevorzugt 320-440°C, ganz besonders bevorzugt 340-410°C, unter adiabatischen Bedingungen gearbeitet wird, wobei der Katalysator mit einem Kreisgas angeströmt wird, das pro Mol Nitrogruppen 3-150 Mol Wasserstoff, 2-100 Mol aromatische Aminogruppen und pro Mol aromatischer Aminogruppen 2-6 Mol, bevorzugt 2,1-4 Mol, besonders bevorzugt 2,2-3 Mol Wasser enthält, die pro Zeitheinheit gebildete Menge Amin und Wasser auskondensiert wird und das restliche Kreisgas, gegebenenfalls nach Abtrennung eines Reinigungsstromes, recyclisiert und mit verdampftem Nitroaromat und mit Frischwasserstoff angereichert wird.

2. Verfahren nach Anspruch 1, dadurch gekennzeichnet, daß Nitroaromaten der Formel 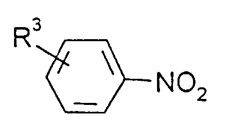 eingesetzt werden, in denen
R³ Wasserstoff, Methyl oder Ethyl bedeutet,
und bevorzugt Nitrobenzol eingesetzt wird.

3. Verfahren nach Anspruch 1, dadurch gekennzeichnet, daß die Reaktion in 1-10, bevorzugt 1-5, besonders bevorzugt 1-3 hintereinandergeschalteten Reaktoren durchgeführt wird, von denen jeder Reaktor durch maximal 5, bevorzugt maximal 3, besonders bevorzugt maximal 2 parallel geschaltete Reakoren ersetzt werden kann.

4. Verfahren nach 3, dadurch gekennzeichnet, daß nach jedem Reaktor die adiabatisch angestaute Reaktionswärme zur Dampfgewinnung eingesetzt wird.

5. Verfahren nach 3, dadurch gekennzeichnet, daß vor jedem Reaktor erneut Nitroaromat dem Reaktionsgemisch zugefügt wird und unabhängig hiervon vor der Nitroaromat-Zugabe ein Teil des Kreisstromes ausgeschleust werden kann, um gebildetes Amin und Wasser auszukondensieren.

6. Verfahren nach Anspruch 1, dadurch gekennzeichnet, daß die Durchdringungstiefe des Katalyatorbettes bei 1 cm - 5 m, bevorzugt 5 cm - 2 m, besonders bevorzugt 10 cm - 1 m, ganz besonders bevorzugt 30 - 60 cm beträgt.

7. Verfahren nach Anspruch 1, dadurch gekennzeichnet, daß das den Katalysator anströmende Kreisgas pro Nitrogruppenäquivalent 2,5 - 50 Mol, bevorzugt 3 - 20 Mol, besonders bevorzugt 3,5 - 10 Mol der zu bildenden Aminogruppen und unabhängig hiervon 6 bis 125 Mol, bevorzugt 12-100 Mol, besonders bevorzugt 25-75 Mol Wasserstoff enthält.

8. Verfahren nach Anspruch 1, dadurch gekennzeichnet, daß ein Palladium auf α-Al₂O₃ enthaltender Katalysator eingesetzt wird, der 1-100 g, bevorzugt 1-60 g, besonders bevorzugt 1-40 g, ganz besonders bevorzugt 1-20 g Pd pro Liter α-Al₂O₃ enthält, das bevorzugt schalenförmig niedergeschlagen ist, wobei der Katalysator zusätzlich Vanadin und Blei enthalten kann und bei einer maximalen Temperatur von 500°C, bevorzugt 480°C, besonders bevorzugt 460°C, ganz besonders bevorzugt 440°C betrieben wird.

9. Verfahren nach Anspruch 1, dadurch gekennzeichnet, daß ein Palladium auf Kohleträgern enthaltender Katalysator eingesetzt wird, dessen Träger eine BET-Oberfläche von 0,2-10 m²/g aufweist, dessen Pd-Gehalt 0,001-1,5 Gew.-% beträgt, dessen Pd-Menge zu 0-40 Relativ-% durch eines oder mehrere Metalle aus der Grupe von Rh, Ir und Ru ersetzt ist, der zusätzlich einen Gehalt von 0,1-2 Gew.-%, bevorzugt 0,1-1 Gew.-% an einer schwefelhaltigen oder phosphorhaltigen, bevorzugt phosphorhaltigen Verbindung, gerechnet als Schwefel oder Phosphor, haben kann, wobei alle Gewichtsprozente auf das Gesamtgewicht des Katalysators bezogen sind, und der bei einer maximalen Temperatur von 480°C, bevorzugt 460°C, besonders bevorzugt 440°C betrieben wird.

10. Verfahren nach Anspruch 1-9, dadurch gekennzeichnet, daß der Katalysator mit 0,1-20 g Nitroaromat pro ml Katalysator pro Stunde, bevorzugt mit 0,2-15 g/ml·h, besonders bevorzugt 0,5-10 g/ml·h, ganz besonders bevorzugt 1-5 g/ml·belastet wird.

## Claims

1. A process for producing aromatic amines of formula 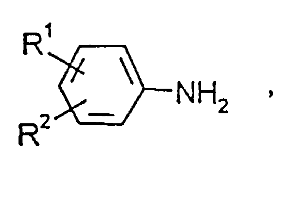 wherein
R¹ and R², independently of each other, represent hydrogen, methyl or ethyl,
and R¹ may in addition represent amino,
by the hydrogenation of aromatic nitro compounds of formula 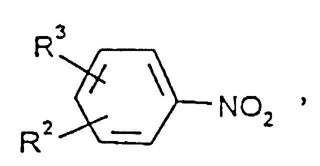 wherein
R² and R³ independently of each other, represent hydrogen, methyl or ethyl, and R³ may in addition represent nitro,
in the gas phase with H₂ over fixed catalysts, characterised in that it is operated at a pressure of 2 to 50 bar, preferably 3-25 bar, most preferably 4-18 bar, and at a temperature in the range 250-500°C, preierably 290-480°C, more preferably 320-440°C, most preferably 340-410°C, under adiabatic conditions, wherein a circulating gas flows over the catalyst, which circulating gas contains 3-150 moles of hydrogen and 2-100 moles of aromatic amino groups per mole of nitro groups, and contains 2-6 moles, preferably 2.14 moles, most preferably 2.2-3 moles, of water per mole of aromatic amino groups, the amount of amine and water formed per unit time is condensed out and the residual circulating gas, optionally after the separation of a purification stream, is recycled and is enriched with vaporised aromatic nitro compound and with fresh hydrogen.

2. A process according to claim 1, characterised in that aromatic nitro compounds of formula 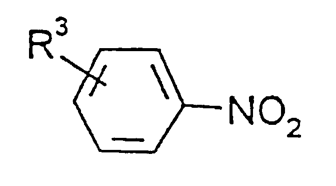 are used, wherein
R³ represents hydrogen, methyl or ethyl,
and nitrobenzene is preferably used.

3. A process according to claim 1, characterised in that the reaction is conducted in 1-10, preferably 1-5, most preferably 1-3 reactors connected in series, each reactor of which may be replaced by a maximum of 5, preferably by a maximum of 3, most preferably by a maximum of 2 reactors connected in parallel.

4. A process according to claim 3, characterised in that downstream of each reactor the adiabatically accumulated heat of reaction is used for the generation of steam.

5. A process according to claim 3, characterised in that fresh aromatic nitro compound is added to the reaction mixture upstream of each reactor and, independently of this, part of the circulating stream can be separated off upstream of the addition of aromatic nitro compound in order to condense out the amine and water formed.

6. A process according to claim 1, characterised in that the penetration depth of the catalyst bed is 1 cm - 5 m, preferably 5 cm - 2 m, more preferably 10 cm - 1 m, most preferably 30 - 60 cm.

7. A process according to claim 1, characterised in that, per nitro group equivalent, the circulating gas flowing over the catalyst contains 2.5 - 50 moles, preferably 3 - 20 moles, most preferably 3.5 - 10 moles, of the amino groups to be formed, and independently thereof contains 6 to 125 moles, preferably 12-100 moles, most preferably 25-75 moles, of hydrogen.

8. A process according to claim 1, characterised in that a catalyst containing palladium on α-Al₂O₃ is used, which contains 1-100 g, preferably 1-60 g, more preferably 1-40 g, most preferably 1-20 g Pd per litre of α-Al₂O₃, which is preferably deposited in the form of a skin, wherein the catalyst may additionally contain vanadium and lead and is operated at a maximum temperature of 500°C, preferably 480°C, more preferably 460°C, most preferably 440°C.

9. A process according to claim 1, characterised in that a catalyst is used which contains palladium on carbon supports, the support of which has a BET specific surface of 0.2-10 m²/g, the Pd content of which is 0.001-1.5 weight %, 0-40 relative % of the amount of Pd of which is replaced by one or more metals of the group comprising Rh, Ir and Ru, which catalyst in addition may have a content of 0.1-2 weight %, preferably 0.1-1 weight %, calculated as sulphur or phosphorus, of a compound containing sulphur or phosphorus, preferably a compound containing phosphorus, wherein all percentages by weight are based on the total weight of the catalyst, and which is operated at a maximum temperature of 480°C, preferably 460°C, most preferably 440°C.

10. A process according to claims 1-9, characterised in that the catalyst is loaded with 0.1-20 g of aromatic nitro compound per ml of catalyst per hour, preferably with 0.2-15 g/ml.hour, more preferably with 0.5-10 g/ml.hour, most preferably with 1-5 g/ml.hour.

## Revendications

1. Procédé pour la préparation d'amines aromatiques de la formule 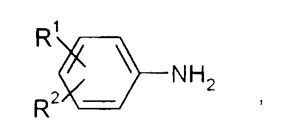 dans laquelle
R¹ et R² représentent indépendamment un atome d'hydrogène, un groupe méthyle ou éthyle, et R¹ peut en outre représenter un groupe amino,
par hydrogénation de composés nitro-aromatiques de la formule 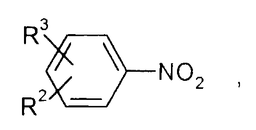 dans laquelle
R² et R³ représentent indépendamment un atome d'hydrogène, un groupe méthyle ou éthyle, et R³ peut en outre représenter un groupe nitro,
en phase gazeuse avec H₂ sur des catalyseurs fixes, caractérisé en ce que l'on travaille à une pression de 2 à 50 bar, de préférence de 3-25 bar, encore mieux de 4-18 bar, et à une température dans le domaine de 250-500°C, de préférence 290-480°C, encore mieux 320-440°C, bien mieux encore 340-410°C, dans des conditions adiabatiques, le catalyseur étant parcouru par un gaz en circulation qui contient par mol de groupes nitro 3-150 mol d'hydrogène, 2-100 mol de groupes amino aromatiques et par mol de groupes amino aromatiques 2-6 mol, de préférence 2,1-4 mol, encore mieux 2,2-3 mol d'eau, la quantité formée par unité de temps d'amine et d'eau est éliminée par condensation et le gaz résiduel en circulation est éventuellement, après séparation d'un courant de purification, recyclé et enrichi avec du composé nitro-aromatique évaporé et avec de l'hydrogène frais.

2. Procédé selon la revendication 1, caractérisé en ce qu'on utilise des composés nitro-aromatiques de la formule 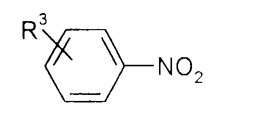 dans laquelle
R³ représente un atome d'hydrogène, un groupe méthyle ou éthyle, et on utilise de préférence le nitrobenzène.

3. Procédé selon la revendication 1, caractérisé en ce que l'on réalise la réaction dans 1-10, de préférence 1-5, encore mieux 1-3 réacteurs raccordés les uns après les autres, chaque réacteur parmi ceux-ci peut être remplacé par au maximum 5, de préférence au maximum 3, encore mieux au maximum 2 réacteurs raccordés en parallèle.

4. Procédé selon la revendication 3, caractérisé en ce que l'on utilise après chaque réacteur la chaleur de réaction adiabatiquement retenue pour une récupération de vapeur.

5. Procédé selon la revendication 3, caractérisé en ce que l'on ajoute au mélange réactionnel à nouveau du composé nitro-aromatique avant chaque réacteur et en ce que l'on peut indépendamment de ceci évacuer avant l'addition du composé nitro-aromatique une partie du courant en circulation pour éliminer l'amine formée et l'eau par condensation.

6. Procédé selon la revendication 1, caractérisé en ce que la profondeur de pénétration du lit de catalyseur est de 1 cm-5 m, de préférence de 5 cm-2 m, encore mieux de 10 cm-1 m, bien mieux encore de 30-60 cm.

7. Procédé selon la revendication 1, caractérisé en ce que le gaz en circulation parcourant le catalyseur contient par équivalent de groupes nitro 2,5-50 mol, de préférence 3-20 mol, encore mieux 3,5-10 mol des groupes amino à former et indépendamment de ceci de 6 à 125 mol, de préférence 12-100 mol, encore mieux 25-75 mol d'hydrogène.

8. Procédé selon la revendication 1, caractérisé en ce que l'on utilise un catalyseur contenant du palladium sur α-Al₂O₃, qui contient 1-100 g, de préférence 1-60g, encore mieux 1-40 g, bien mieux encore 1-20 g de Pd par litre d'α-Al₂O₃, lequel est de préférence déposé dans la forme de pastilles, le catalyseur pouvant en outre contenir du vanadium et du plomb et fonctionnant à une température maximale de 500°C, de préférence de 480°C, encore mieux de 460°C, bien mieux encore de 440°C.

9. Procédé selon la revendication 1, caractérisé en ce que l'on utilise un catalyseur contenant du palladium sur des supports carbonés, dont le support présente une surface spécifique BET de 0,2-10 m²/g, dont la teneur en Pd est de 0,001-1,5% en poids, dont la quantité de Pd est remplacée jusqu'à 0-40% relatifs par un ou plusieurs métaux choisis parmi Rh, Ir et Ru, qui peut en outre présenter une teneur de 0,1-2% en poids, de préférence de 0,1-1% en poids d'un composé contenant du soufre ou du phosphore, de préférence contenant du phosphore, calculée en tant que soufre ou phosphore, tous les pour-cent en poids étant rapportés au poids total du catalyseur, et qui fonctionne à une température maximale de 480°C, de préférence de 460°C, encore mieux de 440°C.

10. Procédé selon la revendication 1-9, caractérisé en ce que l'on charge le catalyseur avec 0,1-20 g de composé nitro-aromatique par ml de catalyseur par heure, de préférence avec 0,2-15 g/ml.h, encore mieux avec 0,5-10 g/ml.h, bien mieux encore avec 1-5 g/ml.h.
